(19) **Europäisches Patentamt / European Patent Office / Office européen des brevets**

(11) **EP 4 570 275 A1**

(12) **EUROPEAN PATENT APPLICATION**

(43) Date of publication:
**18.06.2025 Bulletin 2025/25**

(21) Application number: **23307221.4**

(22) Date of filing: **15.12.2023**

(51) International Patent Classification (IPC):
**A61L 27/04** (2006.01)   **A61L 27/06** (2006.01)
**A61L 27/56** (2006.01)

(52) Cooperative Patent Classification (CPC):
**A61L 27/06; A61L 27/047; A61L 27/56;**
A61L 2400/18; A61L 2430/12

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **ANTHOGYR**
  **74700 Sallanches (FR)**
• **Università Degli Studi Di Torino**
  **10124 Torino (IT)**

(72) Inventors:
• **COURTOIS, Nicolas**
  **74190 PASSY (FR)**
• **DOUEST, Yohan Eric**
  **74380 BONNE (FR)**
• **TIWARI, Kirti**
  **10126 TORINO (IT)**
• **RIZZI, Paola**
  **10131 TORINO (IT)**
• **SCAGLIONE, Federico**
  **10155 TORINO (IT)**

(74) Representative: **Plasseraud IP**
  **104 Rue de Richelieu**
  **CS92104**
  **75080 Paris Cedex 02 (FR)**

(54) **PROCESS FOR PREPARING A MEDICAL DEVICE OR MEDICAL COMPONENT**

(57)    The present invention relates to a process for preparing a medical device or medical component, which comprises a material body comprising an amorphous alloy. The process comprises the steps of

(a) providing a starting material body comprising an amorphous alloy containing

i. a main alloy component selected from the group consisting of titanium copper (TiCu) and zirconium copper (ZrCu), and

ii. one or more secondary alloy components comprising at least one noble metal selected from the group consisting of ruthenium (Ru), rhodium (Rh), palladium (Pd), silver (Ag), osmium (Os), iridium (Ir), platinum (Pt), gold (Au), and combinations thereof, and

(b) subjecting the starting material body to an etching treatment using an etching solution, which dissolves at least one of the alloying elements of the main alloy component of i) at a higher dissolution rate than the at least one noble metal of the secondary alloy component of ii).

Fig. 3

**Description**

The present invention relates to a process for preparing a medical device or medical component according to the preamble of claim 1 as well as to a medical device or medical component prepared by the process.

Medical devices or medical components, such as surgical devices or implants, are typically made of a material, which is biocompatible and exhibits mechanical properties meeting the demanding requirements of the device or component.

Dental implants, for example, are typically made of pure titanium or of a ceramic material, in particular a zirconia-based ceramic. In addition, dental implants made of a titanium alloy (such as a titanium zirconium alloy or a Ti-6Al-4V alloy) have been successfully introduced into the market owed to the material's excellent combination of high strength, high corrosion resistance and sufficient biocompatibility.

Despite the excellent mechanical properties of titanium alloys currently in use, further downsizing of dental implants as well as other medical devices or components is limited due to the associated reduction in stability.

To meet the need of modern surgery for further downsizing medical devices or components without compromising their mechanical properties to an unacceptable extent, amorphous metals, and in particular amorphous alloys such as Bulk Metallic Glasses, have recently gained increasing attention.

For example, CN110464497A relates to a dental implant made of a titanium-based amorphous metal, which is characterized by including: 30-75 parts of titanium, 0-25 parts of zirconium, 0-45 parts of copper, 0-20 parts of silicon, 0-10 parts of iron, 0-10 parts of zinc, 0-5 parts of silver, and 0-15 parts of palladium.

Similarly, CN110464498A relates to a zirconium-based amorphous metal dental implant, which is characterized by including: 30-65 parts of zirconium, 0-25 parts of titanium, 0-45 parts of copper, 0-20 parts of aluminum, 0-20 parts of silicon, 0-10 parts of iron, 0-10 parts of zinc, and 0-5 parts of palladium.

Amorphous metals of the composition disclosed in CN110464497A have been found to have the potential of exhibiting a compressive yield strength and a fatigue resistance much higher than that of Ti-6Al-4V. In addition, these materials have been found to present a high biocompatibility. Thus, the material represents an interesting candidate for applications where a downsizing of an item is aimed at but where a decrease in the mechanical properties must be avoided or at least kept low.

Specifically, $Ti_{40}Zr_{10}Cu_{36}Pd_{14}$ Bulk Metallic Glass (BMG) has been reported to be attractive for future biomedical applications thanks to its high glass forming ability, the absence of toxic elements and its good mechanical properties (see A. Liens et al., "On the Potential of Bulk Metallic Glasses for Dental Implantology: Case Study on Ti40Zr10Cu36Pd14", Materials 2018, 11, 249)). The article of A. Liens et al. also reports on the higher corrosion potential of the respective BMG compared to Ti-6Al-4V alloy, indicating that more energy is required to initiate the corrosion reaction for the BMG.

However, the article also reports on the Ti-based glassy (amorphous) alloy to suffer from pitting corrosion. This unwanted effect is explained in the article referenced above by the presence of copper oxide in the passive layer surface of the BMG and the finding that copper drastically decreases the corrosion resistance of metallic alloys and metallic glasses.

Although the detrimental effects of the material suffering from pitting corrosion are not fully understood yet, it would in view of the material's use for a medical device or component be desirable to reduce or even eliminate this effect while maintaining the desirable properties in terms of mechanical stability and biocompatibility.

In view of the drawbacks mentioned above, the object of the present invention is thus to provide a process for preparing a medical device or component, which in terms of mechanical properties and biocompatibility has the favorable properties of an amorphous alloy, in particular a Ti-based BMG such as $Ti_{40}Zr_{10}Cu_{36}Pd_{14}$, but which simultaneously exhibits a reduced tendency to suffer from pitting corrosion.

This object is solved by the subject matter of claim 1. Preferred embodiments of the present invention are defined in the dependent claims.

According to claim 1, the present invention thus relates to a process for preparing a medical device or medical component comprising a body, which comprises a material body comprising an amorphous alloy.

The process comprises the steps of:

(a) providing a starting material body comprising an amorphous alloy containing

i. a main alloy component selected from the group consisting of titanium copper (TiCu) and zirconium copper (ZrCu), and
ii. one or more secondary alloy components comprising at least one noble metal selected from the group consisting of ruthenium (Ru), rhodium (Rh), palladium (Pd), silver (Ag), osmium (Os), iridium (Ir), platinum (Pt), gold (Au), and combinations thereof, and

(b) subjecting the starting material body to an etching treatment using an etching solution, which dissolves at least one of the alloying elements of the main alloy component of i) at a higher dissolution rate than the at least one noble metal of the secondary alloy component of ii).

Thus, the etching solution provides for a preferential dissolution of titanium or zirconium or, as the case may be, of copper (as alloying elements of the main alloy component of i) over the noble metal(s) of the secondary alloy component of ii) .

According to a preferred embodiment, the etching solution dissolves the at least one noble metal of ii) at a lower dissolution rate than all of the remaining alloy components. Specifically, the at least one noble metal of ii) is not dissolved at all or only to an unsubstantial degree.

According to a specific embodiment of the invention, in which the one or more secondary alloy components of ii) comprise palladium (Pd) and at least one noble metal selected from the group consisting of ruthenium (Ru), rhodium (Rh), silver (Ag), osmium (Os), iridium (Ir), platinum (Pt), gold (Au), and combinations thereof, the etching solution primarily dissolves titanium and/or zirconium as alloying elements of the main alloy component i).

According to yet another specific embodiment, in which the one or more secondary alloy components of ii) comprise no palladium and at least one noble metal selected from the group consisting of ruthenium (Ru), rhodium (Rh), silver (Ag), osmium (Os), iridium (Ir), platinum (Pt), gold (Au), and combinations thereof, the etching solution primarily dissolves copper as alloying element of the main alloy component i). In other words, if no palladium is contained in the amorphous alloy, copper is dissolved preferentially over the other alloying elements of the main alloy component i), namely over titanium and/or zirconium, as well as over the other noble metals of the secondary alloy components ii).

In the course of the present invention, it has been found that by the specific etching treatment of the present invention, a surface region is obtained, the material of which containing a higher proportion of the noble metal component of the secondary alloy component of ii) as compared to the material of the remainder of the material body. This surface region has been found to be crystalline, at least in its outermost area.

In terms of corrosion resistance, this modified surface region has been found to exhibit similar properties as a passivation layer (or a pseudo-passive layer), thus resulting in a substantially reduced tendency of the device or component to suffer from pitting corrosion. (In this regard, pseudo-passivation is characterized by a moderate increase in current density when increasing potential.)

Ultimately, the process of the invention thus allows to obtain a medical device or component, which exhibits exceptional mechanical properties, and which remains stable even if kept in a corrosive environment. This in turn makes it possible to further downsize existing medical devices or components, which despite their reduced dimensions exhibit sufficient mechanical properties and stability over time.

In addition to the above findings, it was further found that by the etching treatment of step (b), the high biocompatibility of the amorphous alloy is not negatively affected. Specifically, a very low hemolysis percentage of both red blood cells and whole blood was determined for the medical device or component of the invention, which emphasizes its suitability for an invasive procedure, in which it comes into contact with blood and body tissue. In accordance with this finding, fibroblast activity was found not to be negatively affected or even slightly improved by the treatment according to the present invention, and the low cytotoxicity of the amorphous alloy was not increased by the treatment.

Still further, the medical device or component has been found to exhibit anti-bacterial activity, both being evidenced by an increased OH-radical release and a decreased biofilm formation in a respective biofilm study. This further emphasizes the suitability of the material in view of its use for a medical device or component, which comes into direct contact with the human body.

Moreover, it was found in the context of the present invention that the etching treatment leads to a roughening of the surface of the device or component, in particular to a porous surface region. This is particularly relevant in view of a potential use of the device or component in a dental implant system, more particularly as a dental implant or dental implant abutment, since there is evidence that a more pronounced roughness is associated with improved interaction of the surface with the surrounding tissue. Thus, a component of a dental implant system can be achieved, which exhibits improved osseointegrative properties and/or improved soft tissue interaction capabilities.

In addition to obtaining an increased corrosion resistance and improved bone and soft tissue interaction capabilities, the process of the present invention further brings about a change in colour of the starting material, and in particular results in a goldish colour. This can be advantageous in some applications, in particular in view of enhancing the aesthetic appearance of the device or component. Specifically, a goldish color is of particular interest in view of the transmucosal region of a dental implant system (implant-soft tissue interface), *e.g.* for the abutment of a dental implant system. In addition, due to the associated brightness and reflectivity, the goldish colour obtained can also be advantageously used to facilitate intra-oral scanning, and the device or component of the present invention thus specifically also relates to such uses.

According to a particularly preferred embodiment of the present invention, the material body comprising the amorphous alloy used is in the form of a Bulk Metallic Glass (or BMG), an amorphous alloy ribbon or an amorphous alloy film. The term "Bulk Metallic Glass" or "BMG" as used in the context of the present invention relates to an amorphous alloy (synonymous to a metallic glass) in bulk form. Specifically, the term "in bulk form" has in this regard the meaning that the amorphous alloy can be produced in a fully dense and amorphous rod having a diameter or thickness of at least 100 $\mu$m, preferably at least 1 mm. In distinction, an amorphous alloy ribbon relates to a body of smaller thickness (typically in a range from 10 $\mu$m to 100 $\mu$m), whereas an amorphous alloy film has a thickness, which is smaller than the one of an amorphous alloy ribbon and typically is less than 10 $\mu$m. Thin films (such as the ones deposited on a surface of a substrate or core) typically have a thickness of 500 nm at most.

For the purpose of the present invention, an amorphous alloy containing titanium and copper as the main alloy components is particularly preferred, since it offers an exceptional specific strength, a high corrosion resistance, a high hardness, a low Young's modulus, as well as a ductility and processing capabilities, which are particularly advantageous for medical devices and components. In this regard, it is further preferred that the titanium copper-based amorphous alloy optionally further contains palladium (Pd) and zirconium (Zr) as secondary alloy component, as this material has been found to particularly suitable for biomedical applications, primarily owed to the absence of toxic elements, such as nickel (Ni) or beryllium (Be). In addition, amorphous alloys of this system have been found to exhibit a glass forming ability high enough to cast a completely amorphous phase *e.g.* dental implants and dental instruments used in oral implantology without compromising their mechanical properties.

With regard to the titanium-copper based amorphous alloy mentioned above optionally containing Pd and Zr as secondary alloy component, it is further preferred that the amorphous alloy further contains at least one tertiary alloy component selected from the group consisting of iron (Fe), gallium (Ga), tin (Sn), silicon (Si), yttrium (Y), silver (Ag), scandium (Sc), sulfur (S), niobium (Nb), hafnium (Hf), zinc (Zn), tantalum (Ta), and mixtures thereof.

More specifically, the amorphous alloy contains 15-55 weight-% of titanium, 1-45 weight-% of copper, 0-30 weight-% of palladium, 0-30 weight-% of zirconium, 0-10 weight-% of silicon, 0-20 weight-% of iron, 0-10 weight-% of zinc and 0-20 weight-% of silver, based on 100 weight-% of the total amount of the amorphous alloy.

In this regard, it is further preferred that the amorphous alloy contains

20-45 weight-%, preferably 25-35 weight-%, of titanium,
15-45 weight-%, preferably 30-45 weight-%, of copper,
5-30 weight-%, preferably 10-20 weight-% of zirconium,
0-30 weight-%, preferably 0-25 weight-% of palladium,
0-15 weight-%, preferably 0-10 weight-% of tin,
0-2 weight-%, preferably 0-1 weight-% of silicon,
0-10 weight-%, preferably 0-5 weight-% of iron,
and 0-15 weight-%, preferably 0-10 weight-% of silver,

based on 100 weight-% of the total amount of the amorphous alloy.

Most preferably, the amorphous alloy is of the composition $Ti_{40}Cu_{36}Zr_{10}Pd_{14}$ (or $Ti_{40}Zr_{10}Cu_{36}Pd_{14}$), whereby the suffixes relate to the atomic percentages of the respective components. This material has been found to exhibit particularly good mechanical properties, in particular a much higher strength and a much lower Young's modulus compared to a Ti-6Al-4V alloy currently in use. The particular suitability of this specific material for the purpose of the present invention will be discussed in detail in the context of the working example below.

Alternatively, the amorphous alloy can be of the composition $Ti_{40}Cu_{40}Zr_{11}Fe_3Sn_3Ag_3$, again with the suffixes relating to the atomic percentages of the respective components. Also this material and its particular suitability for the purpose of the present invention is subject of the working examples discussed below.

With regard to step (b) of the process of the present invention, it has been found that a very efficient etching treatment is achieved when using an etching solution comprising $NH_4$ and $H_2O_2$, which thus relates to a particularly preferred embodiment of the present invention. This preferred etching solution can further comprise $H_2O$ to reduce the kinetic of the reaction, if needed. As regards the percentages and ratios of the components, it is preferred that the volume percentage of each of $NH_4$ and $H_2O_2$ in the etching solution is from 25 to 75 vol.-%. According to a particularly preferred embodiment, the etching solution is a mixture comprising $NH_4$ and $H_2O_2$ with a volume ratio of about 1:1. If water is to be added, the etching solution can in particular be a mixture of $NH_4$, $H_2O_2$ and $H_2O$ with a volume ratio of about 5:1:4 to about 5:4:1, such as 5:1:1, 5:2:1, 5:3:1, 5:4:1, 5:1:2, 5:1:3, 5:1:4. In one embodiment, the volume ration is preferably 5:4:1. In yet another embodiment, the volume ration preferably is 5:1:4.

The kinetic of the reaction can further be adjusted to the respective needs by appropriately setting the temperature of the treatment. According to a straightforward and safe embodiment of the process, the etching treatment is carried out at room temperature.

The duration of the etching depends on the temperature chosen and on the targeted dimension of the modified surface region. In the context of the invention, it has been found that modification of the surface region owed to the differential dissolution rate of the alloying elements occurs within the first minutes of the etching treatment. According to a preferred embodiment, the etching treatment is thus carried out at room temperature for at least 5 minutes, preferably at least 15 minutes, more preferably at least 30 minutes. According to a further preferred embodiment, the etching treatment can last for several hours.

Apart from the process described above, the present invention relates according to a further aspect to a medical device or medical component prepared by the process. Hence, the device or component according to this aspect of the invention comprises a material body comprising an amorphous alloy, which comprises:

I. a main alloy component selected from the group consisting of titanium copper (TiCu) and zirconium copper (ZrCu), and

II. one or more secondary alloy components comprising at least one noble metal selected from the group consisting of ruthenium (Ru), rhodium (Rh), palladium (Pd), silver (Ag), osmium (Os), iridium (Ir), platinum (Pt), gold (Au), and combinations thereof.

According to the invention, the material body comprises a surface region which has an increased proportion of the at least one noble metal of the secondary alloy component of ii) as compared to the remainder of the material body.

Referring to the process of the invention disclosed above, this surface region is obtained by the selective or preferential dissolution of titanium or zirconium or of copper, as the case may be (depending on whether the secondary alloy component of ii) contains palladium or not). Hence, the term "remainder of the material body" relates to the part of the material body extending from a depth of the material body in a direction away from its surface. More specifically, the depth relates in this regard to an area of the material body not accessible to the etching solution.

According to a specific embodiment, the device or component of the present invention comprises a core or substrate and a surface region at least partially surrounding the core or substrate. Thereby, the core or substrate can be made of an amorphous alloy, and specifically relate to the amorphous alloy of the starting material body in its state before treatments. Alternatively, it is also conceivable that the core or substrate is made of a crystalline material, such as a metal, *e.g.* titanium or a titanium alloy such as Ti6Al4V, on which the surface region referred to above is coated. In view of a coating of the core or substrate, e.g. by deposition of the amorphous alloy, it is further conceivable to modify the surface of the core or substrate by a roughening treatment, such as by sandblast, laser treatment or machining, in order to improve adherence of the coating.

As discussed above, the medical device or component of the present invention exhibits a substantially reduced tendency to suffer from pitting corrosion. Owed to the improved corrosion resistance obtained (having similar properties as a passivation layer), the device or component has the advantage to remain corrosion resistant even after a long-term exposure to a humid and warm environment.

As also discussed above, the etching treatment of the process described above leads to roughening of the surface. Specifically, a porosity is formed by the selective dissolution of the alloy components other than the noble metal(s) contained in the amorphous alloy.

More specifically, the surface region of the device or component preferably contains holes and/or pores having an average size of 5 to 30 nm These holes or pores are particularly relevant for devices or components which are destined to interact with the surrounding tissue at their location of use. This is specifically the case for the device or component being a dental implant or a dental implant abutment, for which owed to the surface topography defined above improved osseointegrative properties and/or improved soft tissue interaction capabilities are achieved.

According to a specific embodiment, the surface has a topography defined by a BET surface area in a range of from 2 to 3 $m^2/g$, in particular of 2.17 $m^2/g$ (+/- 0.09). Measurement of the BET surface area (according to the Brunauer-Emmett-Teller (BET) theory) is known to the skilled person and relates to physical adsorption of gas molecules on a solid surface, thus reflecting the specific surface area of the respective surface portion of the device or component.

The use of the device or component as a dental implant or dental implant abutment is further preferred from the point of view that due to the high stability of the amorphous alloy used according to the present invention smaller implant or abutment sizes can be realized than what is feasible with conventional metals. However, apart from the dental implant and dental implant abutment mentioned above, the present invention encompasses any type of medical device or component, and in particular any surgical and dental devices, and components thereof.

Hence, the medical device or medical component is preferably a surgical device or a dental implant, prosthesis or abutment, preferably a dental implant supported prosthesis or abutment, more preferably a screw for use in a dental implant supported prosthesis or abutment. The medical device or component can, however, also be a surgical or a dental instrument, preferably a dental instrument, more preferably a dental driving tool, or a handpiece for use in surgical or dental procedures, in particular a handpiece for dental procedures, or a motor for use in surgical or dental procedures, in particular a motor for use in dental procedures.

**EXAMPLES**

The disclosure is further illustrated by the following examples, which are not to be construed as limiting this disclosure in scope or spirit but are to be understood as illustrating certain embodiments. Unless otherwise noted, reagents and solvents were used as received from commercial suppliers.

In the context of these examples, reference is in particular made to the attached figures, of which

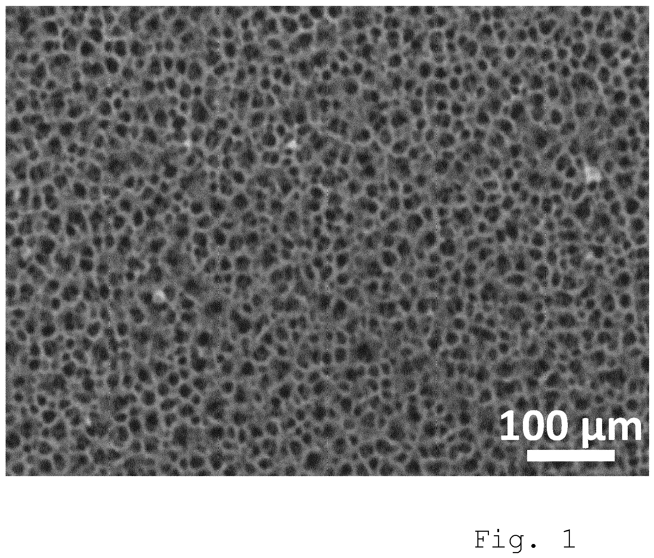

Fig. 1      shows an SEM image of the topography of a component of $Ti_{40}Zr_{10}Cu_{36}Pd_{14}$ BMG obtained by the process of the present invention after 1 hour of treatment at room temperature using a mixture comprising $NH_4$ and $H_2O_2$ (volume ratio 1:1);

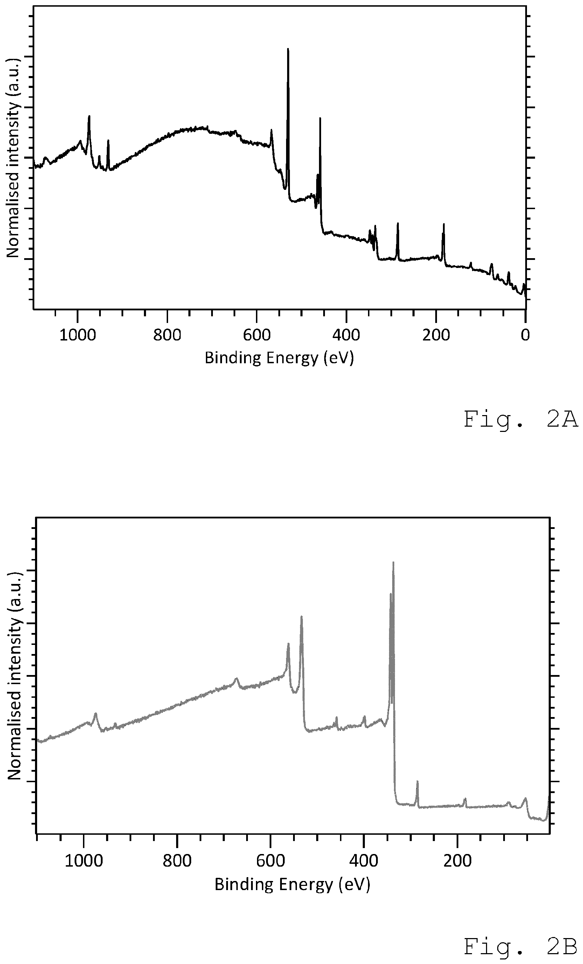

Fig. 2      shows XPS spectra measured on amorphous alloy (BMG) discs of $Ti_{40}Zr_{10}Cu_{36}Pd_{14}$ after 1 hour of treatment at room temperature using a mixture comprising $NH_4$ and $H_2O_2$ (volume ratio of 1:1) as an etching solution for a first sample (Fig. 1B), as an etching solution for a second sample, in comparison to an untreated reference sample (Fig. 1A);

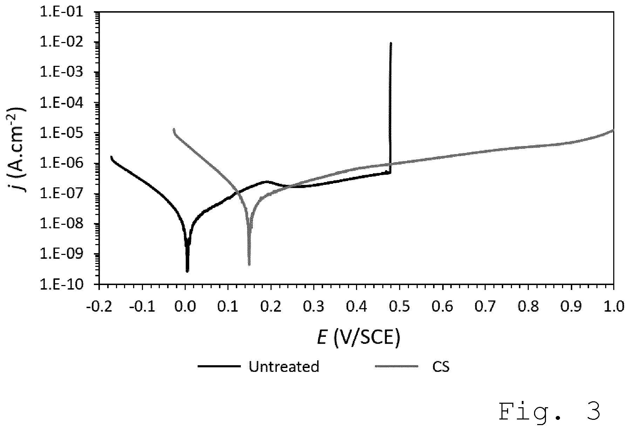

Fig. 3      shows the anodic and cathodic polarization curves of a body made of an amorphous alloy of the composition $Ti_{40}Zr_{10}Cu_{36}Pd_{14}$ and treated according to the present invention ("CS", using a 1:1 $NH_4/H_2O_2$ etching solution for 1 hour) with the polarization being carried out in a 0.9% w/v NaCl solution at pH 7.4 and 37°C in comparison to the polarization curve measured for an untreated body;

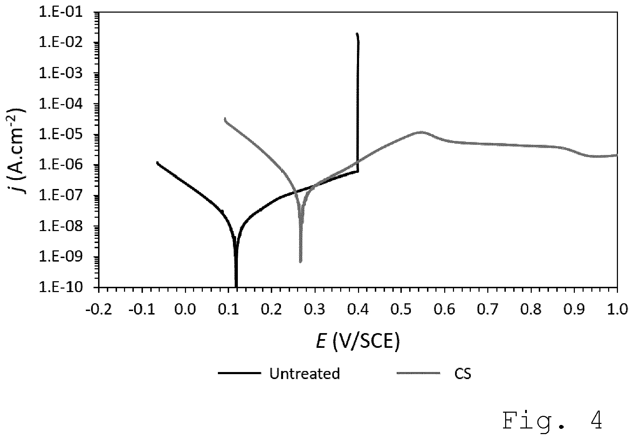

Fig. 4      shows the anodic and cathodic polarization curves of a body composed and treated as defined above for Fig. 3 with the polarization being carried out in a 0.9% w/v NaCl solution (further containing 1% w/v lactic acid) at pH 2.3 and 37°C in comparison to the polarization curve measured for an untreated body;

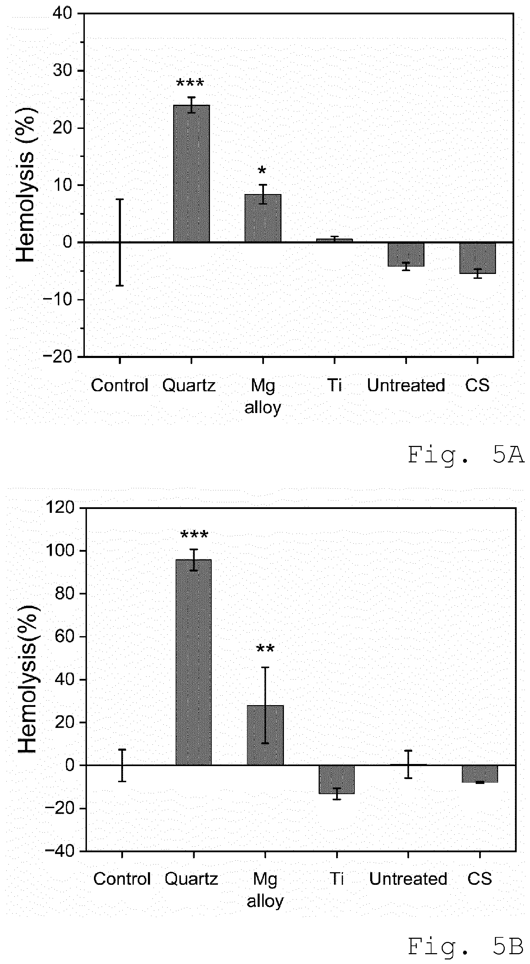

Fig. 5      relates to a diagram indicating the hemolysis percentage of whole blood (Fig. 5A) and red blood cells (Fig. 5B) on a sample obtained according to the process of the present invention (based on $Ti_{40}Zr_{10}Cu_{36}Pd_{14}$) using a concentrated etching solution (CS) as defined above in comparison to an untreated sample as well as to comparative samples made of $SiO_2$, Mg alloy and commercially pure titanium (Cp-Ti), respectively;

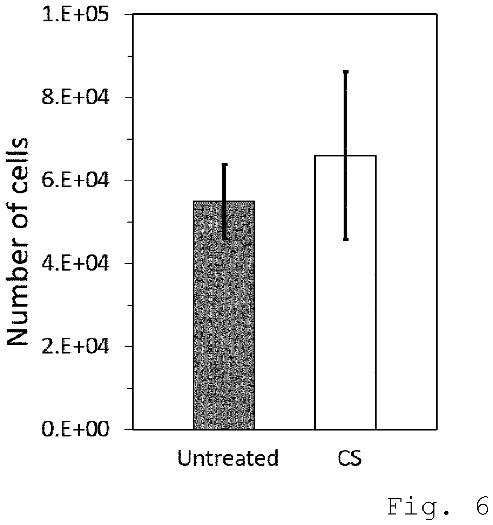

Fig. 6      shows a graphical representation of the average number of HGF-1 cells (human gingiva fibroblast cells) attached to the surface treated according to the present invention (based on the $Ti_{40}Zr_{10}Cu_{36}Pd_{14}$ BMG disc referred to in Fig. 2) determined by nucleocounting, in comparison to the average number of cells attached an untreated reference sample;

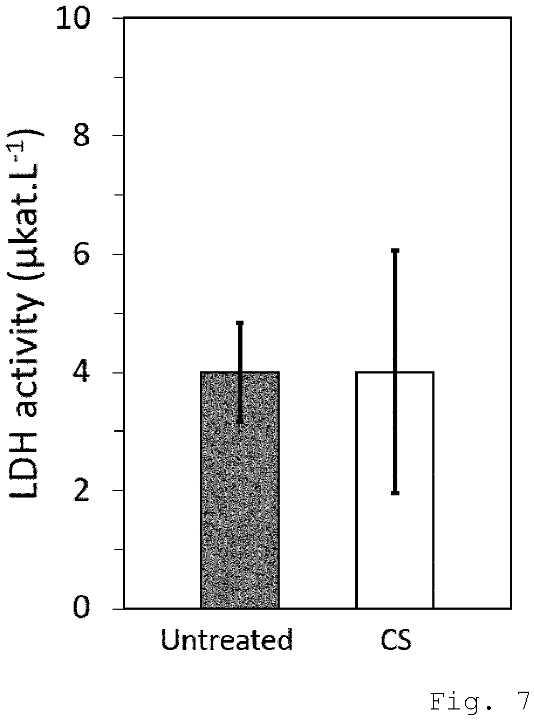

Fig. 7      shows a graphical representation of the average lactate dehydrogenase (LDH) activity of HGF-1 cells after having been in contact with the surface treated according to the present invention in comparison to an untreated reference sample;

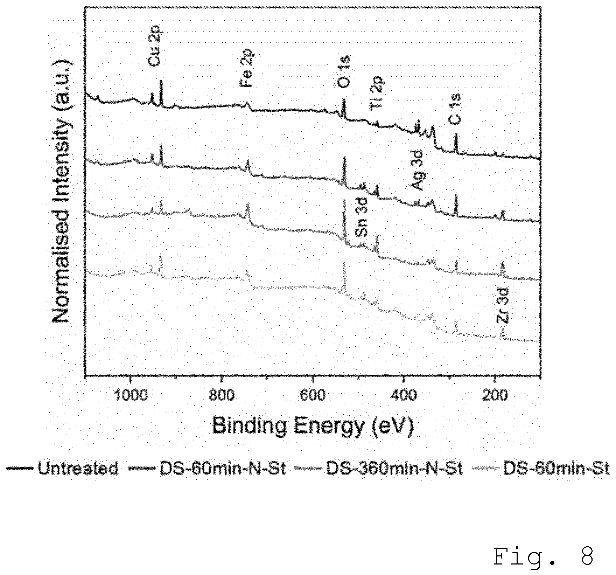

Fig. 8      shows XPS spectra measured on amorphous alloy ribbons of $Ti_{40}Cu_{40}Zr_{11}Fe_3Sn_3Ag_3$ after 1 hour and 6 hours of treatment using a mixture of $NH_4$, $H_2O_2$ and $H_2O$ ("DS"; volume ratio of 5:1:4) as an etching solution without stirring and after 1 hour of treatment with the same etching solution under stirring, compared to an untreated reference sample;

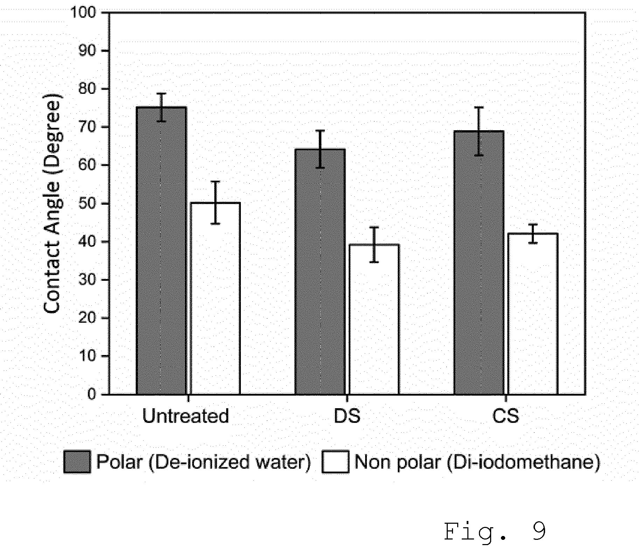

Fig. 9      shows a graphical representation of the advancing contact angle measured for a first sample treated with an etching solution of $NH_4$ and $H_2O_2$ (volume ratio of 1:1) and for a second sample treated with an etching solution of $NH_4$, $H_2O_2$ and $H_2O$ (volume ratio of 5:1:4) as well as for an untreated comparative sample, when being contacted with de-ionized water (for a first wettability measurement) and di-iodomethane (for a second wettability measurement);

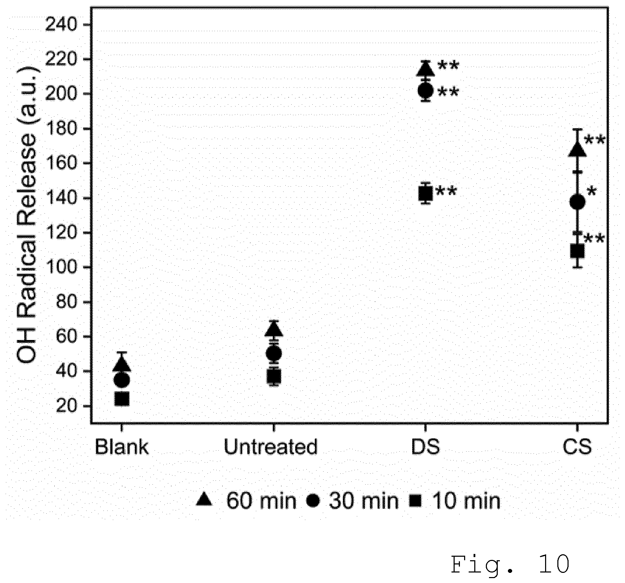

Fig. 10      relates to a graphical representation of the release of OH radicals determined by EPR (electron paramagnetic resonance) Spectroscopy on an amorphous alloy ribbon of $Ti_{40}Cu_{40}Zr_{11}Fe_3Sn_3Ag_3$ treated with an etching solution of $NH_4$ and $H_2O_2$ (volume ratio of 1:1) for a first sample and an etching solution of $NH_4$, $H_2O_2$ and $H_2O$ (volume ratio of 5:1:4) for a second sample, as compared to an untreated ribbon and a blank, for three different treatment times;

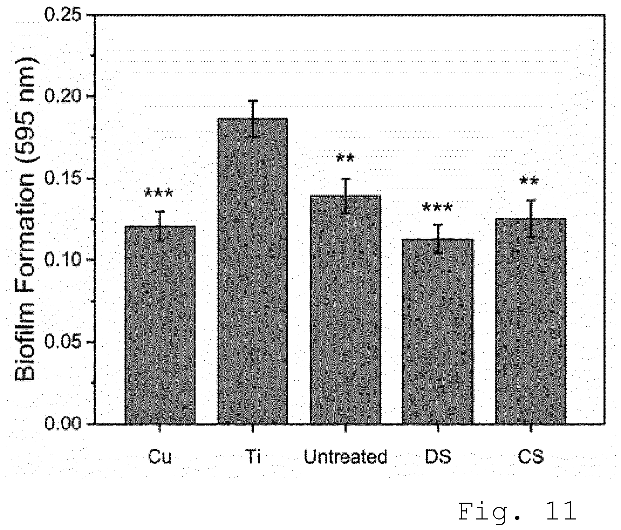

Fig. 11      relates to a graphical representation of the degree of bacterial film bioformation (*pseudomonas aeruginosa*) on the samples referred to in Fig. 10 as further compared to the respective data obtained for a sample of Cu and of Cp-Ti; and

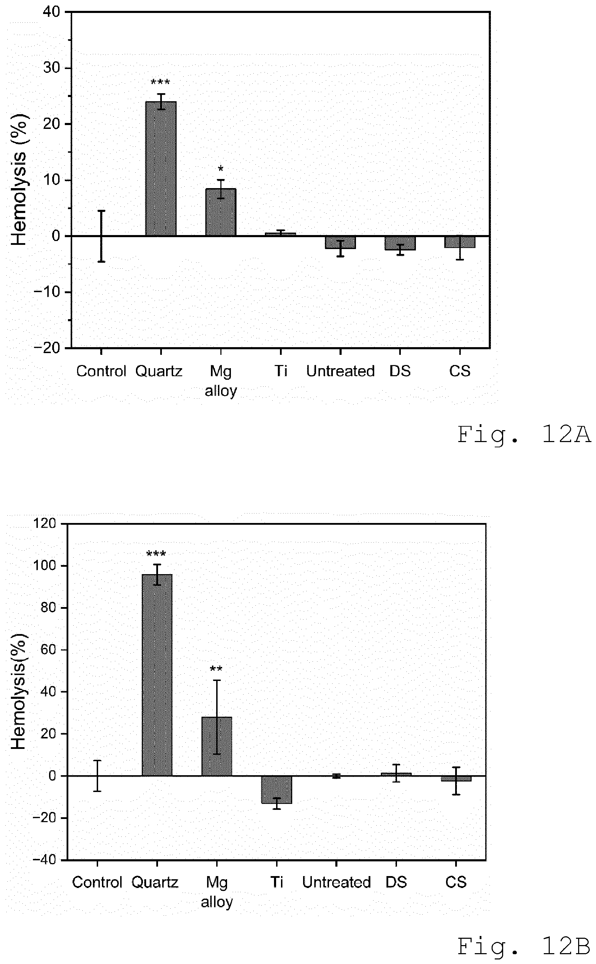

Fig. 12      relates to the graphical representation of the hemolysis of defibrinated whole blood of sheep (Fig. 12A) and of the purified red blood cells obtained therefrom (Fig. 12B) for the samples shown in Fig. 10 in further comparison to the respective hemolysis values determined for quartz particles, Mg alloy and Cp-Ti.

## Experimental details

### Preparation of samples

Master alloy ingots with a targeted composition of $Ti_{40}Zn_{10}Cu_{36}Pd_{14}$ (in atomic %), or $Ti_{29}Zr_{14}Cu_{35}Pd_{23}$ (weight %), and $Ti_{40}Cu_{40}Zr_{11}Fe_3Sn_3Ag_3$ (in atomic %), or $Ti_{30}Cu_{40}Zr_{16}Fe_3Sn_6Ag_5$ (weight %) were prepared by arc melting (Edmund Buhler AM-200) pure elements (purity > 99.5%) under a Ti-gettered argon atmosphere. The master alloys were arc melted at least 3 times to ensure chemical homogeneity.

$Ti_{40}Zr_{10}Cu_{36}Pd_{14}$ alloys were melted by induction heating and cast into 3.4 mm-diameter rods and 25x15x2 mm$^2$ plates by injection-casting into water-cooled mold. The rods and plates were further cut into 0.1 mm-thick disks and 15x10x2 mm$^3$ plates. The samples were polished down to 1 $\mu$m using silicon carbide papers with grains of #1200 and #2500 grits and micron-sized (3 to 1 $\mu$m) diamond suspension.

$Ti_{40}Cu_{40}Zr_{11}Fe_3Sn_3Ag_3$ alloys were melt-spun (Melt Spinner SC) to produce amorphous alloy ribbons using boron nitride (BN) crucible. The alloys were melted using induction heating; molten liquid at 1100°C was ejected with an over pressure $\Delta$p 0.3 bar on a rotating copper wheel at a speed of 30 m/s in an argon atmosphere at 1 bar. The as-quenched ribbons were 4 mm wide and 50 $\mu$m thick.

Prior to the etching treatment, the samples were cleaned in ultrasonic bath of acetone and ethanol. Then the sample were immersed in the etching solution containing $NH_4$ and $H_2O_2$, eventually with $H_2O$. After the treatment, the samples were dried at ambient air.

### SEM

The surface of the treated sample was observed using a Zeiss FEG-SEM Supra 55 VP in secondary electron mode set at an acceleration voltage of 5kV and working distance of 5mm.

### XPS

For the $Ti_{40}Zr_{10}Cu_{36}Pd_{14}$ samples, XPS measurements were carried out using a PHI 5000 Versaprobe II (ULVAC Inc.) equipment with a monochromatized $AlK\alpha$ excitation (1486.6 eV) on an area of 200 $\mu$m of diameter. Survey spectra were recorded from 1100 to 0 eV and post-treated on the software CasaXPS.For the $Ti_{40}Cu_{40}Zr_{11}Fe_3Sn_3Ag_3$ samples, XPS measurements were carried out using an Argon ions (SPECS ion gun) with 100 mm hemispherical analyser. Non-monochromatic $Mg-K\alpha$ X-ray source was used at 400 W and 15 kV. Satellite subtraction was performed for all measurements with charge correction performed on carbon at 284.8 eV.

### Measurement of corrosion resistance

The corrosion performances of the sample bodies were investigated by electrochemical measurements in a three-electrode glass cell using a Gamry instrument 600+ potentiostat (GAMRY Instruments) with a graphite rod as counter electrode and a saturated calomel electrode (SCE) as reference electrode (potential $E_{SCE}$ = 268 mV vs Standard Hydrogen Electrode potential at 25 °C). The working electrode consisted of a sample body connected to a copper wire on one of its circular faces. Only one treated circular face of the sample body was in contact with the electrolyte solution, the other part of the sample body being insulated by waterproof sheath.

The electrochemical tests were performed in two different electrolyte solutions maintained at 37°C by a water circulating temperature control unit: 1) in an aerated and neutral saline solution (pH = 7.4) composed of 0.9 weight/volume percentage concentration (w/v%) of sodium chloride (NaCl) as recommended in the ISO 10271 standard and 2) in an aerated acidic saline solution (pH = 2.3) composed of 0.9 w/v% NaCl + 1 w/v% lactic acid.

The electrochemical tests consisted of open circuit potential (OCP) measurements during 6H to reach a steady state and were followed by potentiodynamic polarization measurements. The polarization curves parameters were set to a scan rate of 0.17 mV/s (millivolt per seconds), from -0.2 V vs SCE to +1 V vs SCE.

### Hemolysis assessment

Hemolysis studies were carried out by defibrinated sheep blood (whole blood, Microbiol Diagnostic) and purified red blood cells (RBCs) from sheep blood in Alsever's solution (Thermo Fisher). Each medium (i.e., whole blood and RBC's) were diluted in phosphate buffered saline (PBS) solution with a volume ratio 4:5 (blood:PBS). The absorbance (Abs) of was adjusted to 0.9 - 1.0 by adding PBS or blood. A positive control was prepared by using 10 mL of ultrapure water (Merck-Millipore) and 0.2 mL diluted media (total volume=10.2 mL). Similarly, a negative control with PBS and diluted media was prepared.

Samples were sterilized using 70 % ethanol followed by washing with ultrapure water and PBS. The samples were immersed separately in glass vials containing 10.2 mL whole blood and 10.2 mL RBC media, the vials were incubated in a horizontal thermostatic shaker for 60 min at 37°C. For comparison, quartz particles (SiO$_2$, Min-U-Sil 5, U.S Silica, 200 cm2/ml, specific surface area: 5 m2/g)) and magnesium alloy (WE43B, Goodfellow) were used as positive-reference materials (i.e., materials triggering haemolysis) while commercially pure titanium (Cp-Ti) was used as negative-reference material (i.e., no influence on the haemolysis).

After immersion, the supernatant containing potentially released haemoglobin was separated from by centrifugation at 500 G (Centrifuge Rotina 380R) for 5 min and the absorbance was measured with a UVS 900 Lite Spectrophotometer device at a wavelength of 540 nm. The measurements were repeated three time for each sample. The percentage of hemolysis of test samples was calculated as follow:

$$\% \text{ Hemolysis} = \frac{\text{Abs(test)} - \text{Abs(negative control)}}{\text{Abs(positive control)} - \text{Abs(negative control)}} \times 100$$

(eq. 1)

### Assessment of fibroblast activity

HGF-1 (Human Gingiva Fibroblast) cell line (ATCC CRL-2014, Manassas, USA) was cultured in DMEM (Dulbecco's Modified Eagle's Medium) supplemented with 10% fetal bovine serum and 1% penicillin/streptomycin solution (Gibco Life Technologies, Carlsbad, California, USA). The cells were grown in 75 cm$^2$ culture flasks (Sarstedt, Nümbrecht, Germany) at 37°C in a humidified incubator with 5% CO2. Prior to cell seeding, disk-shaped materials from Ti$_{40}$Zr$_{10}$Cu$_{36}$Pd$_{14}$ BMG disks were sterilized by immersing them in 70% ethanol for 20 minutes, followed by three rinses with sterile water and air-drying. After 24 hours of incubation on the samples, HGF-1 cells were detached using trypsin (Gibco Life Technologies, Waltham, USA) and the number of viable adhered cells on the surface was quantified using a NucleoCounter® NC-200TM system (ChemoMetec A/S, Lillerød, Denmark), following the manufacturer's instructions. In addition, the LDH (lactate dehydrogenase) activity was measured from the supernatant using CyQUANT™ LDH Cytotoxicity Assay (Thermo Fisher Scientific, Roskilde, Denmark), following the manufacturer's protocol. Absorbance was measured at 490 nm every minute for 30 minutes using a FLUOstar Omega Microplate reader (BMG LABTECH, Ortenberg, Germany).

### Wettability study; Contact angle measurement

Wettability studies were carried out using Theta lite optical tensiometer (Biolin Scientific) in sessile drop. To understand the wettability and surface free energy (SFE) of the sample surface, polar and non-polar solvents were used. The contact angle was measured after 1 second of placing droplet of 3 $\mu$L volume of de-ionized water (polar) and di-iodomethane (non-polar), using oneAttension software. The surface free energy of the pseudo-dealloyed samples was calculated using Young's equation and Owen, Wendt, Rabel and Fowkes (OWRK) method. The measurement was recorded with a minimum of 3 droplets on 3 samples for each group (n=9).

### Measuring Biofilm

To investigate biofilm formation, gram-negative *Pseudomonas aeruginosa* (PAO1) was utilized. The bacterial inoculum was grown in Lysogeny broth (Sigma-Aldrich, LB media: 1% tryptone, 0.5% yeast extract, 0.5% NaCl) in DI H$_2$O. Prior to bacterial inoculation, the samples (0.4 $\times$ 2 cm) underwent sterilization by immersion in 70% ethanol, and each study was conducted with three biological replicates and two technical replicates.

Overnight cultures of *P. aeruginosa* were grown in 15 mL of LB (lysogeny broth) media at 37 °C. Bacteria were quantified by diluting an aliquot 1:10 in fresh media and the optical density (OD) was measured at 600 nm using a spectrophotometer (Spectramax m384). Cultures were then equalized to 0.1 OD600 cultures (10$^7$ CFU (Colony Forming Unit)/mL) in preparation for inoculation. The samples were then incubated without shaking in 1 mL of the equalized bacterial culture for 24 hours at 37 °C in sterile 24-well, flat-bottomed, tissue culture-treated plates.

Following the incubation period, the samples were transferred to a new well-plate containing phosphate-buffered saline (PBS) solution and rinsed gently three times with PBS to remove non-adhered planktonic bacteria. Subsequently, the samples were dried at 37 °C for 30 minutes. 1% crystal violet (CV) solution (1 mL) was added to each well and left at room temperature for 15 minutes. After staining, the ribbons were transferred to a new well-plate and rinsed three times with PBS and dried at 37 °C for 30 minutes. 1 mL of elution buffer (80% ethanol and 20% acetone) was then added to the wells and left at room temperature for 15 minutes. Afterwards, 1 mL of this elution was transferred to an optically clear plastic cuvette, and the OD of the solution was measured at 595 nm using elution buffer as blank providing a quantitative measurement of biofilm biomass.

**EPR Spectroscopy**

EPR Spectroscopy for determining the degree in release of OH radicals was performed by spin trapping technique coupled with electron paramagnetic resonance spectroscopy (EPR) was employed. Cleaned metallic samples with $0.4 \times 1$ cm size were immersed in 1 ml of phosphate buffer (PB, 0.5M, pH 7.4, Sigma-Aldrich, Milan, Italy) containing 0.04 M DMPO (5,5-dimethyl-1-pyrroline-1-oxide, Cayman Chemical Company, Ann Arbor, Michigan) as spin trap molecule, and freshly prepared 0.2 M $H_2O_2$ as target molecule. The samples were incubated in the solution at 37 °C, while being continuously stirred using a thermostatic shaker in darkness. After 10, 30, and 60 minutes, 50 $\mu$L of the supernatant were collected in a capillary, and electron paramagnetic resonance spectroscopy (EPR) spectra were recorded utilizing a Miniscope MS100 spectrometer (Magnettech, Berlin, Germany) at a microwave power level of 10 mW, a modulation of 1 Gauss (G), scan range of 120 G, and a center field at 3355 G. A blank control without samples was prepared. The number of radicals released are proportional to the intensity of the EPR signal. To compare the amount of hydroxyl radicals produced by the samples, the signals were double integrated using Origin 2023 software and results were reported as arbitrary units. The measurements were conducted in duplicate.

**_Results_**

As shown by the SEM image according to Fig. 1, the treatment according to step b) of the present invention leads to the formation of nano-sized features, in particular of pores and holes in the nanometer range, thus giving clear evidence of selective/preferential etching.

The modification of the surface region obtained by the treatment is further revealed by Fig. 2, showing that the treatment of the sample according to the present invention showed a marked reduction of Ti and O and an increase of Pd (i.e. a noble metal of the secondary alloy component of II) in the surface region of the sample as compared to the untreated sample.

The analysis shows that by the etching treatment of the present invention, the noble metal palladium contained in the amorphous alloy is dissolved at a lower dissolution rate than the remaining alloy components, thus resulting in a surface region, which contains a higher proportion of palladium as compared to the untreated sample. Since the etching treatment according to the present invention, which is limited to the surface region of the starting material body "accessible" to the etching solution, an amorphous alloy body is obtained having in its surface region an increased proportion of palladium as compared to the remainder of the material body.

As shown in Fig. 3 (relating to the corrosion resistance measurement at physiological pH), increasing the potential leads to the sample of the present invention showing a large pseudo-passive region wherein the current density increases moderately. In contrast, the untreated sample showed a sudden increase in current density at a potential value of 0.48 V/SCE, which is characteristic of pitting corrosion.

A similar result in terms of a reduced pitting corrosion susceptibility of the samples of the present invention is shown in Fig. 4 (relating to the corrosion resistance measurement in an acidic environment). According to Fig. 4, a high/large passive plateau with no pitting event was observed for the sample of the present invention, showing that the surface region obtained (functioning as a passivation layer) is also protective in an environment which is both chlorine-containing and acidic-containing.

Both these results (Fig. 3 and Fig. 4) are indicative of a higher corrosion resistance and a reduced susceptibility to pitting corrosion of the samples according to the present invention over the comparative samples.

The samples treated according to the present invention further showed a good hemocompatibility, as shown in Fig. 5. Specifically, the hemolysis percentage of the whole blood determined for the sample according to the present invention was comparable to the values obtained for commercially pure titanium (Cp-Ti) known for its high biocompatibility, and much lower than the one determined for $SiO_2$ and Mg alloy. Similarly, also the hemolysis percentage of the red blood cells (RBC) was significantly lower for the sample according to the present invention as compared to the one determined for $SiO_2$ and Mg alloy. Since the hemolysis percentage of both the whole blood and the RBC determined for the sample of the invention were comparable (or even slightly lower) than the one of untreated sample, the data is indicative of the fact that biocompatibility of the amorphous alloy is not negatively affected by the treatment of the invention.

The suitability of the samples according to the invention for an invasive procedure is further illustrated by Fig. 6, representing the number of cells attached to the surface that has been treated by the inventive process, as compared to an untreated sample. Specifically, nucleocounting revealed that the average number of cells attached to the samples according to the present invention was slightly higher than for the untreated sample, indicating a similar or even higher fibroblast activity of the samples after treatment.

As shown in Fig. 7, LDH activity of cells after having been in contact with the surface treated according to the present invention was similar to the one measured for cells in contact with an untreated sample, showing that the samples of the present invention exhibit a similar (low) cytotoxicity as the untreated sample.

The findings discussed above in the context of Fig. 2, i.e. that the treatment of the present invention results in a surface region containing a higher proportion of the noble metal of secondary alloy component ii) as compared to the untreated sample, is confirmed by the XPS spectra according to Fig. 8, relating a different amorphous alloy, namely $Ti_{40}Cu_{40}Zr_{11}Fe_3Sn_3Ag_3$. Fig. 8 further shows a change of the XPS spectra over time (specifically after 1 hour and 6 hours), which reveals that after 6 hours of etching treatment, the proportion of copper is substantially reduced in the surface region. The spectra also show an increase in the content of titanium (Ti) and oxygen (O) over time, indicative for the formation of titanium oxide in the surface region by a prolonged etching treatment.

For the material of the present invention (referred to in the context of Fig. 8), it was further found that the hydrophilicity is increased, both in wettability studies using de-ionized water and using di-iodomethane as contacting medium. This is reflected by the respective contact angles measured on two samples of the present invention (obtained by an etching treatment using 1:1 $NH_4/H_2O_2$, referred to as CS, and 5:1:4 $NH_4/H_2O_2/H_2O$, referred to as DS) and on an untreated comparative sample, the results of which are shown in Fig. 9. The data obtained by these studies relate to a surface free energy (SFE; in mN/m) of 50.17 for the DS sample and 46.85 for the CS sample, as compared to 40.85 of the untreated sample.

As shown in Fig. 10, OH radical release was found to be markedly increased for the samples according to the present invention, the release increasing with the duration of treatment and being more pronounced for the samples obtained by the treatment with the diluted etching solution 5:1:4 $NH_4/H_2O_2/H_2O$ than with the concentrated etching solution (1:1 $NH_4/H_2O_2$). Hence, the samples of the present invention can boost the production of reactive oxygen species in a cell free system mimicking inflammatory conditions, which can show anti-bacterial activity.

The samples of the present invention exhibiting increased anti-bacterial properties is further confirmed by the results of the biofilm study using a Crystal Violet (CV 1%) assay, which are shown in Fig. 11. According to this study, a significant decrease of the optical density and hence of the biofilm formation (*pseudomonas aeruginosa*) was determined, giving further evidence that the samples according to the invention have anti-bacterial activity and thus can prevent bacteria adhesion and biofilm formation.

As shown in Fig. 12, the samples obtained by the process of the present invention have been shown to be hemocompatible. In other words, the high hemocompatibility of the untreated material has been found to remain unaffected by the treatment of the present invention, as discussed above in the context of Fig. 5 for $Ti_{40}Zr_{10}Cu_{36}Pd_{14}$.

**Claims**

1. A process for preparing a medical device or medical component, which comprises a material body comprising an amorphous alloy, wherein the process comprises the steps of

    (a) providing a starting material body comprising an amorphous alloy containing

        i. a main alloy component selected from the group consisting of titanium copper (TiCu) and zirconium copper (ZrCu), and
        ii. one or more secondary alloy components comprising at least one noble metal selected from the group consisting of ruthenium (Ru), rhodium (Rh), palladium (Pd), silver (Ag), osmium (Os), iridium (Ir), platinum (Pt), gold (Au), and combinations thereof, and

    (b) subjecting the starting material body to an etching treatment using an etching solution, which dissolves at least one of the alloying elements of the main alloy component of i) at a higher dissolution rate than the at least one noble metal of the secondary alloy component of ii).

2. The process according to claim 1, wherein the etching solution dissolves the at least one noble metal of ii) at a lower dissolution rate than all of the remaining alloy components.

3. The process according to any one of the preceding claims, wherein the material body comprising the amorphous alloy is in the form of a Bulk Metallic Glass, an amorphous alloy ribbon or an amorphous alloy film.

4. The process according to any one of the preceding claims, wherein the amorphous alloy contains titanium and copper as the main alloy components and optionally further contains palladium (Pd) and zirconium (Zr) as secondary alloy component.

5. The process according to any of claims 1 to 4, wherein the amorphous alloy further contains at least one tertiary alloy component selected from the group consisting of iron (Fe), gallium (Ga), tin (Sn), silicon (Si), yttrium (Y), silver (Ag), scandium (Sc), sulfur (S), niobium (Nb), hafnium (Hf), zinc (Zn), tantalum (Ta), and mixtures thereof.

6. The process according to claim 5, wherein the amorphous alloy contains 15-55 weight-% of titanium, 1-45 weight-% of copper, 0-30 weight-% of palladium, 0-30 weight-% of zirconium, 0-10 weight-% of silicon, 0-20 weight-% of iron, 0-10 weight-% of zinc and 0-20 weight-% of silver, based on 100 weight-% of the total amount of the amorphous alloy.

7. The process according to claim 6, wherein the amorphous alloy contains

>20-45 weight-%, preferably 25-35 weight-%, of titanium,
>15-45 weight-%, preferably 30-45 weight-%, of copper,
>5-30 weight-%, preferably 10-20 weight-% of zirconium,
>0-30 weight-%, preferably 0-25 weight-% of palladium,
>0-15 weight-%, preferably 0-10 weight-% of tin,
>0-2 weight-%, preferably 0-1 weight-% of silicon,
>0-10 weight-%, preferably 0-5 weight-% of iron,
>and 0-15 weight-%, preferably 0-10 weight-% of silver,
>based on 100 weight-% of the total amount of the amorphous alloy.

8. The process according to any of claims 1 to 7, wherein the amorphous alloy is of the composition $Ti_{40}Zr_{10}Cu_{36}Pd_{14}$, the suffixes relating to the atomic percentages of the respective components.

9. The process according to any one of the preceding claims, wherein the etching solution comprises $NH_4$ and $H_2O_2$.

10. The process according to claim 9, wherein in the etching solution the volume percentage of each of $NH_4$ and $H_2O_2$ is from 25 to 75 vol.-%, and preferably the etching solution is a mixture comprising $NH_4$ and $H_2O_2$ with a volume ratio of about 1:1.

11. The process according to any one of the preceding claims, wherein the etching treatment is carried out at room temperature for at least 5 minutes, preferably at least 15 minutes, more preferably at least 30 minutes.

12. A medical device or medical component prepared by the process according to any of the preceding claims, the device or component comprising a material body comprising an amorphous alloy, which comprises

>I. a main alloy component selected from the group consisting of titanium copper (TiCu) and zirconium copper (ZrCu), and
>II. one or more secondary alloy components comprising at least one noble metal selected from the group consisting of ruthenium (Ru), rhodium (Rh), palladium (Pd), silver (Ag), osmium (Os), iridium (Ir), platinum (Pt), gold (Au), and combinations thereof,

wherein the material body comprises a surface region having an increased proportion of the at least one noble metal of the secondary alloy component of II) as compared to the remainder of the material body.

13. The medical device or component according to claim 12, wherein the surface region contains holes and/or pores having an average size of 5 to 30 nm.

14. The medical device or component according to any of claims 12 or 13, wherein it is a part of a dental implant system, and in particular is a dental implant or a dental implant abutment.

Fig. 1

Fig. 2A

Fig. 2B

Fig. 3

Fig. 4

Fig. 5A

Fig. 5B

Fig. 6

Fig. 7

Fig. 8

Fig. 9

Fig. 10

Fig. 11

Fig. 12A

Fig. 12B

Europäisches
Patentamt

European
Patent Office

Office européen
des brevets

## EUROPEAN SEARCH REPORT

**Application Number**

EP 23 30 7221

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 110 464 497 B (UNIV PEKING SCHOOL STOMATOLOGY) 20 August 2021 (2021-08-20) * claim 1 * | 1-11 | INV. A61L27/04 A61L27/06 A61L27/56 |
| A | PETRE FLAVIU GOSTIN ET AL: "In Situ Synchrotron X-Ray Diffraction Characterization of Corrosion Products of a Ti-Based Metallic Glass for Implant Applications", ADVANCED HEALTHCARE MATERIALS, WILEY - V C H VERLAG GMBH & CO. KGAA, DE, vol. 7, no. 21, 16 September 2018 (2018-09-16), page n/a, XP072468195, ISSN: 2192-2640, DOI: 10.1002/ADHM.201800338 * abstract * | 1-14 | |
| X | LIENS ALÉTHÉA ET AL: "Effect of alloying elements on the microstructure and corrosion behavior of TiZr-based bulk metallic glasses", CORROSION SCIENCE, OXFORD, GB, vol. 177, 6 July 2020 (2020-07-06), XP086363894, ISSN: 0010-938X, DOI: 10.1016/J.CORSCI.2020.108854 [retrieved on 2020-07-06] * figure 9 * | 12,14 | TECHNICAL FIELDS SEARCHED (IPC) A61L |

The present search report has been drawn up for all claims

| Place of search | Date of completion of the search | Examiner |
|---|---|---|
| The Hague | 1 May 2024 | Siebum, Bastiaan |

EPO FORM 1503 03.82 (P04C01)

## ANNEX TO THE EUROPEAN SEARCH REPORT
## ON EUROPEAN PATENT APPLICATION NO.

EP 23 30 7221

This annex lists the patent family members relating to the patent documents cited in the above-mentioned European search report.
The members are as contained in the European Patent Office EDP file on
The European Patent Office is in no way liable for these particulars which are merely given for the purpose of information.

01-05-2024

| Patent document cited in search report | Publication date | Patent family member(s) | Publication date |
|---|---|---|---|
| CN 110464497 B | 20-08-2021 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110464497 A

- CN 110464498 A

**Non-patent literature cited in the description**

- **A. LIENS et al.** On the Potential of Bulk Metallic Glasses for Dental Implantology: Case Study on Ti40Zr10Cu36Pd. *Materials*, 2018, vol. 11, 249